# FASCICULE DE BREVET EUROPEEN

(11) **EP 1 851 196 B1**
(45) Date de publication et mention de la délivrance du brevet: **01.12.2010**
(21) Numéro de dépôt: 06709311.2
(22) Date de dépôt: 14.02.2006
(51) Int. Cl.: C07D 201/08, C07D 223/10

(54) **PROCEDE DE FABRICATION DE LACTAMES**
VERFAHREN ZUR HERSTELLUNG VON LACTAMEN
LACTAM PRODUCTION METHOD

(30) Priorité: 22.02.2005 FR 0501761
(43) Date de publication de la demande: 07.11.2007
(73) Titulaire: RHODIA CHIMIE, 93300 Aubervilliers (FR)
(72) Inventeur: LECONTE, Philippe, 68150 Ribeauvillé (FR)
(74) Mandataire: Esson, Jean-Pierre
(86) Numéro de dépôt international: PCT/FR2006/000331
(87) Numéro de publication internationale: WO 2006/090044

(56) Documents cités:
- EP-A- 0 023 751
- WO-A-98/05636
- WO-A-98/37063
- US-A- 4 470 928
- US-A1- 2003 153 749
- US-B1- 6 365 770

## Description

La présente invention concerne un procédé de fabrication de lactames.

Elle concerne plus particulièrement un procédé de fabrication de lactames à partir de composés alkylcyanovalérates obtenus en utilisant comme matières premières notamment des composés mononitriles insaturés, et plus particulièrement des pentènenitriles tels que les 2-, 3-, 4-pentènitriles pris isolément ou en mélange et appelés ci-après PN pour le mélange et respectivement 2PN, 3PN, et 4PN.

L' ε-caprolactame est un composé utilisé préférentiellement pour la fabrication de différents polyamides dont le plus important est le polyamide 6 (PA6) ou polycaproamide.

Plusieurs procédés de synthèse d'ε-caprolactame ont été proposés dont certains sont exploités industriellement depuis de nombreuses années. Le procédé le plus exploité utilise comme matière première du benzène pour fabriquer l'oxime de cyclohexanone comme composé intermédiaire, l'ε-caprolactame étant obtenu par la réaction de réarrangement de Beckmann.

Depuis plusieurs années, il a également été proposé un procédé de fabrication de ε-caprolactame en utilisant comme matière première le butadiène et avec comme composé intermédiaire, l'adiponitrile

Dans ce procédé, l'adiponitrile obtenu par double hydrocyanation du butadiène est partiellement hydrogéné en aminocapronitrile avec production conjointe d'hexaméthylènediamine.

L'aminocapronitrile, après séparation, est hydrolysé et cyclisé en caprolactame soit en phase gazeuse soit en phase liquide, en présence ou non d'un solvant. Ce procédé demande une double hydrocyanation du butadiène et produit conjointement de l'hexamethylènediamine qui doit être valorisée pour l'économie du procédé.

Il est également possible, à partir du butadiène, de réaliser une alkoxycarbonylation pour obtenir le penténoate d'alkyle, puis une hydroformylation suivie d'une amination réductrice en aminocaproate d'alkyle. Ce dernier composé est ensuite cyclisé en caprolactame.

Il est également proposé un procédé de synthèse de caprolactame en utilisant comme matière première du butadiène et comme composé intermédiaire des pentènenitriles (PN) obtenus par une simple hydrocyanation d'une double liaison du butadiène. Dans ce procédé les PN sont convertis en formylvaléronitrile dans une réaction d'hydroformylation en présence d'hydrogène et de monoxyde de carbone.

Ce formylvaléronitrile est ensuite transformé, dans une seconde étape, en alkylcyanovalérate par oxydation et réaction avec un alcool.

Après isolation par, par exemple, distillation, l'alkylcyanovalérate est hydrogéné pour obtenir l'alkylaminocaproate. Le caprolactame est obtenu dans une dernière étape par cyclisation de l'alkylaminocaproate.

Un tel procédé, notamment décrit dans le brevet US 6 365 770, comprend de nombreuses étapes successives nécessitant entre chaque étape une séparation du composé intermédiaire formé.

Ces différentes étapes de séparation entraînent une perte de rendement global du procédé et donc affectent fortement l'économie de celui-ci.

Cet enchaînement de réactions et étapes successives est décrit dans de nombreux brevets et articles comme par exemple le brevet WO 01/96294.

Un des buts de la présente invention est de remédier à ces inconvénients en proposant un procédé qui permet d'améliorer son rendement global et de réduire l'investissement nécessaire pour l'exploitation industrielle de celui-ci.

A cet effet, l'invention propose un procédé de synthèse de caprolactame à partir d'alkylcyanovalérate qui se caractérise en ce qu'il consiste à mettre en contact l'alkylcyanovalérate à l'état gazeux avec de l'hydrogène, en présence de catalyseurs d'hydrogénation et de cyclisation, et à récupérer le flux gazeux contenant le caprolactame formé et à traiter ledit flux, après condensation, pour récupérer le caprolactame.

Le procédé de l'invention permet d'obtenir le caprolactame directement à partir de l'alkylcyanovalérate sans étape d'isolation et de récupération de l'alkylaminocaproate formé in-situ.

De ce fait, l'investissement nécessaire pour l'exploitation de ce procédé est notablement réduit, ainsi que les pertes de produits générées au cours des étapes de séparation de l'alkylaminocaproate ou par des réactions de polymérisation.

Selon un mode de réalisation préféré de l'invention, le procédé s'applique plus particulièrement à la synthèse de l'ε-caprolactame à partir d'un alkylcyanovalérate tel que le méthylcyanovalérate.

Selon une autre caractéristique de l'invention, le traitement du flux gazeux, en sortie de réacteur, consiste à condenser le flux gazeux et à le traiter pour séparer les différents composants et récupérer le caprolactame. A titre d'exemple, le flux condensé peut être traité par des résines échangeuses d'ions. Le milieu obtenu comprenant le caprolactame, après traitement sur résine est distillé en présence d'une base forte pour séparer l'alcool et/ou le solvant et récupérer le caprolactame.

Un tel procédé de traitement d'un milieu de cyclisation pour l'obtention de caprolactame pur est notamment décrit dans le brevet EP 922 027.

Il est également possible sans sortir du cadre de l'invention, d'utiliser tous les procédés connus permettant d'extraire et purifier le caprolactame contenu dans un milieu.

Ainsi, cette extraction et/ou purification peut comprendre des étapes de cristallisation, hydrogénation, oxydation, par exemple

L'alkylcyanovalérate utilisé comme matière première dans le procédé de l'invention peut être obtenu, par exemple, à partir de pentènenitriles par une réaction d'hydroformylation puis oxydation et réaction avec un alcool tel que décrit, à titre indicatif, dans les brevets US 6 365 770, US 5 986 126 et WO 00/56451.

L'alkylcyanovalérate peut également être obtenu par alcoxycarbonylation des pentènenitriles par réaction avec le monoxyde de carbone et un alcool tel que le méthanol. De tels procédés sont décrits dans les brevets WO 01/72697, WO 03/040159 et WO 00/14055.

D'autres procédés de fabrication sont décrits, par exemple, dans l'article de Reppe publié dans Lieb. Ann. Chem. 596 (1995)127, les brevets BE 850113, EP 576976.

De manière générale, tout procédé connu de synthèse d'un alkylcyanovalérate est convenable pour l'invention, comme le procédé utilisant l'hydrolyse enzymatique de l'adiponitrile décrit notamment, dans le brevet WO 97/44318.

Selon l'invention, la transformation de l'alkylcyanovalérate en caprolactame est mise en oeuvre dans un réacteur unique contenant un système catalytique présentant d'une part une activité catalytique pour l'hydrogénation et d'autre part, une activité catalytique pour la réaction de cyclisation.

Selon un mode de réalisation de l'invention, les catalyseurs d'hydrogénation et de cyclisation sont des composants distincts qui sont présents dans le réacteur sous forme de mélange de poudres ou granulés solides, ou sous forme de lits catalytiques notamment quand le réacteur est tubulaire ou sous forme de colonne. Dans ce dernier mode de réalisation, les deux lits catalytiques sont avantageusement disposés de manière successive et adjacente dans le réacteur, le lit de catalyseur d'hydrogénation étant disposé en amont du lit de catalyseur de cyclisation dans le sens du déplacement des gaz ou vapeurs constituant le flux réactionnel. Le réacteur peut également contenir un seul lit catalytique comprenant un mélange des deux catalyseurs.

Dans un autre mode de réalisation de l'invention, le système catalytique unique est constitué par un catalyseur supporté, le support étant avantageusement un catalyseur de cyclisation, les métaux catalysant l'hydrogénation étant déposés ou absorbés sur le dit support.

A titre d'exemple de catalyseurs d'hydrogénation pouvant être utilisés sous forme de mélange ou sous forme de lit catalytique, on peut citer les catalyseurs comprenant comme élément métallique actif le fer, le ruthénium, le rhodium, l'iridium, le palladium, le cobalt, le nickel, le chrome, l'osmium et le platine ou plusieurs éléments de cette liste. Ces métaux peuvent être utilisés sous forme de catalyseurs supportés, ou sous forme massique. De tels catalyseurs sont notamment décrits dans les brevets US2003/0153749 et US 6 365 770.

De manière générale, tous les supports de catalyseurs sont convenables pour réaliser ces catalyseurs d'hydrogénation. Un ou plusieurs métaux sont déposés à la surface de ces supports, notamment sous forme d'oxyde. La quantité de métal sur le support n'est pas critique mais est généralement comprise entre 0,1 % et 50 % en poids par rapport au poids de catalyseur supporté.

Concernant la réaction de cyclisation, les catalyseurs convenables sont des catalyseurs hétérogènes solides tels que ceux décrits, par exemple, dans la demande de brevet européen 1 456 177.

Parmi les catalyseurs décrits dans ce document les oxydes métalliques tels que les alumines ; la silice, ainsi que les zéolithes, les phosphates métalliques tels que, par exemple, les phosphates d'aluminium, phosphates de titane, phosphate de zirconium sont particulièrement convenables pour l'invention.

Comme catalyseurs préférés de cyclisation de l'invention, on citera les alumines poreuses, notamment celles décrites dans les brevets européens n° 0805801 et 1098875.

Selon un mode de réalisation de l'invention, le système catalytique est avantageusement un catalyseur unique qui comprend une activité de catalyse pour la réaction d'hydrogénation et une activité catalytique pour la réaction de cyclisation. Les catalyseurs préférés de l'invention présentant ces activités sont des catalyseurs obtenus par dépôt d'un ou plusieurs éléments métalliques présentant une activité catalytique en hydrogénation et décrits ci-dessus, sur un composé solide correspondant aux catalyseurs de cyclisation décrits ci-dessus. Ainsi, les catalyseurs préférés de l'invention sont les catalyseurs comprenant un oxyde métallique tel que les alumines poreuses décrites ci-dessus sur lequel est déposé au moins un élément métallique catalytiquement actif. Ces catalyseurs peuvent être obtenus par tous les procédés classiques de fabrication des catalyseurs supportés.

Selon l'invention, il est possible de mettre en oeuvre les réactions d'hydrogénation et de cyclisation en présence d'ammoniac et/ou d'eau. Avantageusement, la concentration pondérale en ammoniac et/ou eau dans le milieu réactionnel est comprise entre 5 et 40% Ces réactions sont réalisées à une température comprise entre 200°C et 450°C et avantageusement sous une pression partielle d'hydrogène comprise entre 0,1 et 20 bar

La fabrication du lactame selon le procédé de l'invention peut être mise en oeuvre dans tout réacteur permettant la réaction entre des gaz par passage sur un catalyseur avantageusement à l'état solide.

Ainsi, les réacteurs préférés sont les réacteurs tubulaires ou les réacteurs colonnes qui peuvent comprendre des lits fixes ou fluidisés de catalyseurs.

Le flux de gaz en sortie de réacteur est avantageusement refroidi rapidement pour éviter la formation d'oligomères par polymérisation du lactame.

Le lactame récupéré est ensuite purifié et récupéré selon les procédés de purification connus. Ainsi, dans un mode de réalisation de l'invention, le flux gazeux sortant du réacteur est condensé et refroidi rapidement à une température inférieure à 150°C. Cette étape de condensation et refroidissement est réalisée en un temps compris entre quelques secondes et quelques minutes, l'ammoniac éventuellement présent est ensuite éliminé par distillation. Le milieu résultant comprenant le caprolactame en solution dans l'alcool formé (le méthanol dans le cas du méthylcyanovalérate) ou en milieu hydrométhanolique, est ensuite purifié par traitement sur résines, hydrogénation, oxydation, cristallisation et/ou distillation. Le caprolactame récupéré présente un degré de pureté comparable à celui obtenu par les différents procédés de synthèse connus.

D'autres avantages, détails de l'invention apparaîtront plus clairement au vu des exemples donnés ci-après uniquement à titre d'illustration.

### Exemples 1 à 5

Les essais ont été réalisés dan un réacteur cylindrique composé d'un tube en verre équipé avec des moyens de chauffage électrique, d'une sonde de mesure de la température, d'une entrée et une sortie des gaz et d'un moyen d'introduction du réactif.

Le tube en verre, en position verticale, est remplie successivement en se déplaçant de bas en haut, avec 5 ml de bille de quartz, 4 ml du catalyseur A, 4 ml de catalyseur B et 5 ml de billes de quartz.

Selon les exemples, le catalyseur A est constitué d'un catalyseur d'hydrolyse et le catalyseur B est un catalyseur d'hydrogénation, ou les catalyseurs A et B sont identiques et constituent un catalyseur mixte constitué par un élément métallique déposé sur un support, généralement et préférentiellement de l'alumine.

Le réacteur est chauffé à 300 °C sous un courant d'hydrogène alimenté par le haut du réacteur avec un débit de 2,5 1/h. Au bout d'une heure, la solution méthanolique de cyanovalérate de méthyle (le réactif) est alimenté dans le flux d'hydrogène avec un débit de 2 ml/h. Cette solution contient 60% en poids de cyanovalérate de méthyle.

Les vapeurs recueillies en sortie de réacteur sont condensées et analysées par chromatographie en phase gazeuse en utilisant comme étalon intense du butyl benzène.

Le taux de conversion (TT) du cyanovalérate de méthyle et le rendement (RR) en caprolactame sont calculés à partir des résultats des analyses.

Les résultats obtenus sont rassemblés dans le tableau ci-dessous :

| Ex | Catalyseur | | TT en % | RR en % | |
|---|---|---|---|---|---|
| | A | B | | | |
| 1 | Al₂O₃ * | Ni/Al₂O₃ | 98 | 37 | (1) |
| 2 | Al₂O₃ * | Rh/Al₂O₃ | 100 | 45 | (2) |
| 3 | Pt/Al₂O₃ | Pt/ Al₂O₃ | 93 | 33 | (2) |
| 4 | Al₂O₃ * | Pd/ Al₂O₃ | 100 | 37 | (2) |
| 5 | Al₂O₃ * | Rh/ Al₂O₃ | 86 | 57 | (2), (3) |

| | | | | | |
|---|---|---|---|---|---|
| * Al₂O₃ est une alumine de volume poreux 117 ml/100g et surface spécifique 139 m²/g commercialisée par la société Axens (1) Catalyseur commercialisé par la société Johnson Mattey (2) Catalyseurs commercialisés par la société Engelhard contenant 0,5 % en poids d'élément métallique (3) Le cyanovalérate de méthyle est alimenté sous forme pure (sans solvant) | | | | | |

## Revendications

1. Procédé de synthèse de caprolactame à partir alkylcyanovalérate **caractérisé en ce qu'**il consiste à mettre en contact l'alkylcyanovalérate à l'état gazeux avec de l'hydrogène en présence de catalyseurs d'hydrogénation et de cyclisation et à traiter, après condensation, le flux gazeux contenant le lactame formé, le catalyseur de cyclisation étant une alumine et le catalyseur d'hydrogénation comprenant un élément ou un mélange d'éléments métalliques choisi dans le groupe comprenant comme élément métallique actif le fer, le ruthénium, le rhodium, l'iridium, le palladium, le cobalt, le nickel, le chrome, l'osmium et le platine ou plusieurs métaux de cette liste

2. Procédé selon la revendication 1, **caractérisé en ce que** le catalyseur de cyclisation est choisi dans le groupe comprenant les oxydes métalliques, les zéolithes, les phosphates métalliques.

3. Procédé selon l'une des revendications 1 ou 2, **caractérisé en ce que** les catalyseurs d'hydrogénation et de cyclisation sont mélangés.

4. Procédé selon l'une des revendications 1 à 3, **caractérisé en ce que** le catalyseur d'hydrogénation et le catalyseur de cyclisation sont disposés de manière séparée dans le réacteur pour former deux lits catalytiques successifs.

5. Procédé selon l'une des revendications précédentes, **caractérisé en ce que** l'alkylcyanovalérate est le méthylcyanovalérate.

6. Procédé selon l'une des revendications précédentes **caractérisé en ce que** la réaction est mise en oeuvre à une température comprise entre 200°C et 450°C

7. Procédé selon l'une des revendications précédentes, **caractérisé en ce que** la réaction est mise en oeuvre sous une pression d'hydrogène comprise entre 0,1 et 20 bar.

8. Procédé selon l'une de revendications précédentes, **caractérisé en ce que** le traitement du flux gazeux sortant du réacteur, après condensation, comprend une étape de traitement sur résine échangeuses d'ions ou une distillation en présence d'un acide fort, et une étape de distillation du caprolactame en présence d'une base forte.

9. Procédé selon la revendication 12, **caractérisé en ce que** l'ammoniac, s'il est présent, est extrait du flux gazeux condensé avant l'étape de traitement sur résine échangeuse d'ions ou distillation en présence d'un acide.

## Claims

1. Process for the synthesis of caprolactam from alkyl cyanovalerate, **characterized in that** it consists in bringing the alkyl cyanovalerate, in the gas state, into contact with hydrogen in the presence of hydrogenation and cyclization catalysts and in treating, after condensation, the gas stream comprising the lactam formed, the cyclization catalyst being an alumina and the hydrogenation catalyst comprising a metal element or a mixture of metal elements chosen from the group consisting, as active metal element, of iron, ruthenium, rhodium, iridium, palladium, cobalt, nickel, chromium, osmium and platinum, or several metals from this list.

2. Process according to Claim 1, **characterized in that** the cyclization catalyst is chosen from the group consisting of metal oxides, zeolites and metal phosphates.

3. Process according to either of Claims 1 and 2, **characterized in that** the hydrogenation and cyclization catalysts are mixed.

4. Process according to one of Claims 1 to 3, **characterized in that** the hydrogenation catalyst and the cyclization catalyst are positioned separately in the reactor to form two successive catalytic beds.

5. Process according to one of the preceding claims, **characterized in that** the alkyl cyanovalerate is methyl cyanovalerate.

6. Process according to one of the preceding claims, **characterized in that** the reaction is carried out at a temperature of between 200°C and 450°C.

7. Process according to one of the preceding claims, **characterized in that** the reaction is carried out under a hydrogen pressure of between 0.1 and 20 bar.

8. Process according to one of the preceding claims, **characterized in that** the treatment of the gas stream exiting from the reactor, after condensation, comprises a stage of treatment on ion-exchange resin or a distillation in the presence of a strong acid and a stage of distillation of the caprolactam in the presence of a strong base.

9. Process according to Claim 8, **characterized in that** the ammonia, if it is present, is extracted from the condensed gas stream before the stage of treatment on ion-exchange resin or distillation in the presence of an acid.

## Patentansprüche

1. Verfahren zur Synthese von Caprolactam ausgehend von einem Alkylcyanovalerat, **dadurch gekennzeichnet, dass** man bei dem Verfahren das Alkylcyanovalerat im gasförmigen Zustand mit Wasserstoff in Gegenwart von Hydrierungs- und Zyklisierungskatalysatoren in Kontakt bringt und dass man den Gasstrom, der das gebildete Lactam enthält, nach der Kondensation behandelt, wobei der Zyklisierungskatalysator ein Aluminiumoxid ist und der Hydrierungskatalysator ein metallisches Element oder ein Gemisch von metallischen Elementen umfasst, die aus der Gruppe ausgewählt sind, die als aktives metallisches Element Eisen, Ruthenium, Rhodium, Iridium, Palladium, Kobalt, Nickel, Chrom, Osmium und Platin oder mehrere Metalle aus dieser Auflistung umfasst.

2. Verfahren nach Anspruch 1, **dadurch gekennzeichnet, dass** der Zyklisierungskatalysator aus der Gruppe ausgewählt ist, die Metalloxide, Zeolithe und Metallphosphate umfasst.

3. Verfahren nach einem der Ansprüche 1 oder 2, **dadurch gekennzeichnet, dass** der Hydrierungs- und der Zyklisierungskatalysator gemischt werden.

4. Verfahren nach einem der Ansprüche 1 bis 3, **dadurch gekennzeichnet, dass** der Hydrierungskatalysator und der Zyklisierungskatalysator in dem Reaktor getrennt voneinander platziert werden, derart, dass sie zwei aufeinander folgende Katalysatorbetten bilden.

5. Verfahren nach einem der vorhergehenden Ansprüche, **dadurch gekennzeichnet, dass** es sich bei dem Alkylcyanovalerat um Methylcyanovalerat handelt.

6. Verfahren nach einem der vorhergehenden Ansprüche, **dadurch gekennzeichnet, dass** man die Umsetzung bei einer Temperatur zwischen 200°C und 450°C durchführt.

7. Verfahren nach einem der vorhergehenden Ansprüche, **dadurch gekennzeichnet, dass** man die Umsetzung unter einem Wasserstoffdruck zwischen 0,1 und 20 bar durchführt.

8. Verfahren nach einem der vorhergehenden Ansprüche, **dadurch gekennzeichnet, dass** die Behandlung des Gasstroms, der aus dem Reaktor kommt, nach der Kondensation einen Schritt der Behandlung auf Ionenaustauschharz oder eine Destillation in Gegenwart einer starken Säure und einen Schritt der Destillation des Caprolactams in Gegenwart einer starken Base umfasst.

9. Verfahren nach Anspruch 8, **dadurch gekennzeichnet, dass** Ammoniak, falls vorhanden, aus dem kondensierten Gasstrom vor dem Schritt der Behandlung auf Ionenaustauschharz oder der Destillation in Gegenwart einer Säure extrahiert wird.
